# EUROPEAN PATENT SPECIFICATION

(11) **EP 2 996 764 B1**
(45) Date of publication and mention of the grant of the patent: **19.07.2017**
(21) Application number: 13729613.3
(22) Date of filing: 15.05.2013
(51) Int. Cl.: A61N 1/36, A61H 39/00

(54) **A SYSTEM FOR DECREASING THE BLOOD FLOW OF A TARGETED ORGAN'S ARTERY WITH AN ELECTRICAL STIMULATION**
SYSTEM ZUR VERRINGERUNG DES BLUTFLUSSES DER ARTERIE EINES ZIELORGANS MIT EINER ELEKTRISCHEN STIMULATION
SYSTÈME POUR DIMINUER LE FLUX SANGUIN DE L'ARTÈRE D'UN ORGANE CIBLÉ AU MOYEN D'UNE STIMULATION ÉLECTRIQUE

(43) Date of publication of application: 23.03.2016
(73) Proprietor: KOC Universitesi, 34450 Istanbul (TR)
(72) Inventor: CAKMAK, Yusuf Ozgur, 34310 Istanbul (TR)
(74) Representative: Cayli, Hülya
(86) International application number: PCT/EP2013/060060
(87) International publication number: WO 2014/183790

(56) References cited:
- WO-A1-01/52931
- WO-A1-2010/088914
- WO-A2-2012/058289
- US-A1- 2002 138 116
- US-A1- 2003 114 900
- US-A1- 2004 230 256
- US-A1- 2007 270 917

## Description

### Field of Invention

The present invention relates to a computer-aided internal bleeding severity diagnosis system for medical use, which outputs data displayed for skin electrostimulation map and guides skin placements of the system integrated electrodes which apply blood flow decreasing electrostimulation.

### Prior Art

Current applications to decrease the internal organ bleeding are externally applied foams, clothes or devices that need surgical implantation. Further, they are not organ specific and further devices that are placed into or around the vessels with surgical applications or the devices use mechanical compressions, chemical reactions or local muscle contraction methods.

Examples of such applications are disclosed in US2003114900 A1, US2007055337 A1, WO2010118178 A2, US7226615 B2 and patent application US20120107439 in addition to EP 1703881 A2 and EP 0 957 773 B1.

US2003114900 A1 system comprises means for constant localization (foot) for stimulation area and it is not targeting a specific individual internal organ and a specific effect as decreasing blood flow in an individual internal organ's artery. Further the conventional system with US2003114900 A1 itself cannot provide a specific map for to decrease blood flow in an artery of a specific organ and the system itself lack of calibration and optimization of the decreasing the blood flow in an individual organ. In addition, the system is cumbersome for to be carried easily.

US2007055337 A1 system comprises means for improving blood flow by targeting muscles contractions. In addition, the system uses high voltage for human and it can only be effective when applied to the injured zone.

WO2010118178 A2 system comprises means for modulate blood flow of the uterus with the aid of uterine contractions. The system described in the above USP comprises means for modulating the uterus only.

US7226615 B2 discloses means for foam for external bleedings and needs injections. US Patent application no. 20120107439 entitled as in situ forming hemostatic foam implants comprises means for decreasing bleeding but it needs an intravascular injection. EP 1703881 A2 comprises means for a wound dressing only for the external bleedings. EP 0 957 773 B1 comprises means for intravascular occlusion with a percutaneous catheter directed intravasculary. The system described in the above EP needs intravascular surgical implantation of the device.

PCT Patent Application WO/2009/103298 entitled as intra-abdominal balloon device needs intra-abdominal placement and it only targets to overcome uterine bleeding with the mechanical compression of the vessels.

### Brief Description of Invention

The invention is defined in claim 1. Further aspects and preferred embodiments are defined in the dependent claims. The present invention relates to a system to decrease the blood flow of a targeted organ in case of bleeding or a medical requirement, characterized in that the system comprises an integrated electrostimulator, which comprises electrodes to be placed to the skin of a patient, and a subsystem which comprises at least one first and at least one second input means. The first input means comprises blood pressure measurement and pulse detector unit and the second input means is a device enabling the user to enter the wounded or bleeding area information into the system. Said subsystem determines where the said electrodes are placed according to analyzed blood pressure, pulse and inputted wounded or bleeding area information according to an algorithm embedded in the subsystem, and the subsystem further display where the said electrodes are placed through at least a skin map.

### Object of Invention

It is an object of the present invention to overcome internal bleedings with the aid of an organ based targeting system without a need for a surgery, intravascular applications or a medical professional and to provide an extended time of homeostasis up to medical professional help and/or surgical applications.

### Description of Figures

Figure 1 is a flowchart of stimulator application and electrode placement.
Figure 2 shows an illustration of the system's components application.
Figure 3 shows bilateral placement of the two electrodes placement skin maps for to achieve a 20% blood flow decrease to liver, stomach, kidney, spleen and proximal large intestines.
Figure 4 shows other bilateral placement of the two electrodes placement skin maps or to achieve a 20% blood flow decrease to distal large intestine, testicles (gonads), prostate gland and bladder.
Figure 5 shows bilateral placement of the four electrodes placement skin maps for to achieve a 40% blood flow decrease to distal large intestine, testicles (gonads), prostate gland and bladder.
Figure 6 shows other bilateral placement of the four electrodes placement skin maps for to achieve a 40% blood flow decrease to all abdominal organs.
Figure 7 shows another bilateral placement of the four electrodes placement skin maps for to achieve a 40% blood flow decrease to the liver, stomach, spleen and proximal Large intestines.

The components in said figures are individually referenced as following;

| | |
|---|---|
| Display unit | (10) |
| Electrodes | (11) |
| Wounded or bleeding area | (12) |
| Blood pressure measurement and pulse detector unit | (13) |
| Wireless communication equipment | (14) |

| | |
|---|---|
| L5 dermatome | (a) |
| L4-S2 dermatome | (b) |
| T12 dermatome | (c) |
| T10 dermatome | (d) |

### Detailed Description of invention

Present invention is related to a system comprising an integrated specific electrostimulator to decrease the blood flow to a targeted internal organ in the case of an internal bleeding or a medical requirement such as surgery, The system further comprises a subsystem which comprises at least one first and at least one second input means. The first input means comprises blood pressure measurement and pulse detector unit and the second input means is a device enabling the user to enter the wounded or bleeding area information into the system. Said subsystem determines where the said electrodes are placed according to analyzed blood pressure, pulse and inputted wounded or bleeding area information according to an algorithm embedded in the subsystem, and the subsystem further display where the said electrodes are placed through at least a skin map.

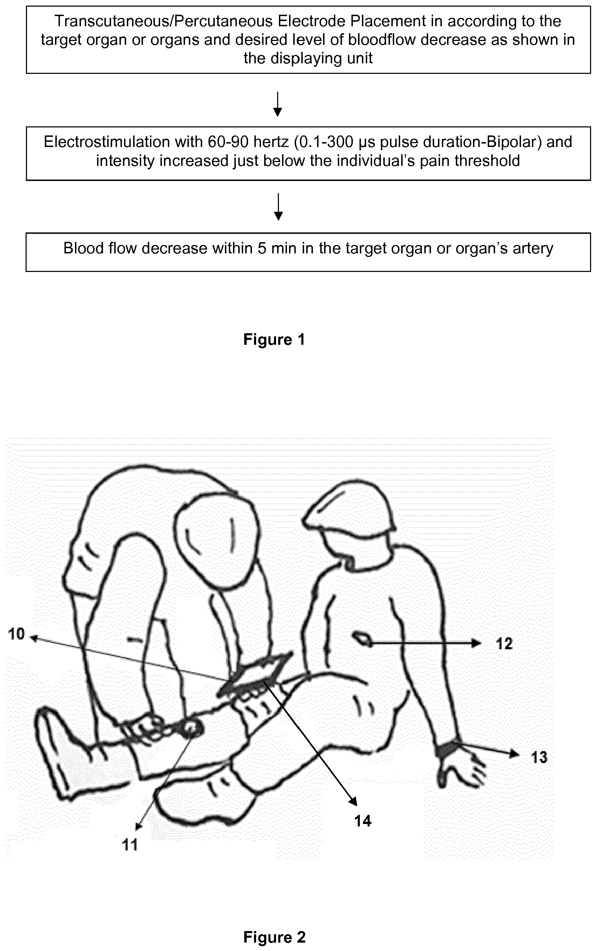

Present invention also relates to a system for decreasing the blood flow of a targeted internal organ's artery with an electrical stimulation (Figure 2). The system of the invention comprises the following steps:
- Transcutaneous/Percutaneous electrode (11) placement in according to the target organ or organs and desired level of blood flow decrease as shown in the display unit (10),
- Electrostimulation with 60-90 hertz (0.1-300 µs (microsecond) pulse duration - bipolar) and intensity increased just below the individual's pain threshold, and
- Blood flow decrease within 5 min in the target organ or organ's artery.

The present invention is related to a handheld system which uses specific combinations of the electrical stimulation (percutaneous and/or transcutaneous) and skin dermatomal (a skin area that is supplied by a nerve) zones to decrease the blood flow of a desired internal organ. Further, said system let the user to calibrate the level of the blood flow decreases of the desired organ's artery in between 20% to 44% percent with specific placement of the electrodes' combinations over the different skin dermatomes in 3-5 minutes.

In addition, the system of the present invention comprises a means for high current blocker for currents exceeding 15mA to overcome skin burning and preferably comprises thermoelectric coolers to cool the skin around the electrodes and avoid burning. Such a function would be useful for example to stimulate in higher currents without a burning in the skin. In the case of using currents exceeding 15 mA the high current blocker has to be switched off. Thermoelectric coolers can be also used at current levels below 15 mA.

The system further comprises an integrated visual and acoustic alarm system for the user to manage the internal bleeding of more than one individual. This would be important, if for example more than one individual is wounded and the same doctor has to care all of them. The system can further comprise communication means such as wireless communication equipment (14) to inform the doctor or first aid personal. The communication means sends attributed data, which contains blood pressure and heart beat data, in a coded form enabling error detection and error correction and making the communication less vulnerable to noise. Such coding techniques are known in the art.

Algorithm of the present invention simply comprises: monitoring the heart rate and blood pressure measured i.e. separately or in combination from the fingers, arm and wrist by blood pressure measurement and pulse detector unit (13), and interpretation of the data reflected as an electrode placement map on the display unit (10) with specific software (Figure 1).

In the present invention, there is provided a computer-aided diagnosis system comprising a subsystem wherein the subsystem has following means:
- first input means for blood pressure and pulse data of an individual with an internal organ bleeding,
- means for storing a plurality of computer-aided diagnosis algorithms and skin map for electrode placement,
- means for selecting, based on the blood pressure and beat data, the power of blood decreasing level in the targeted organ/organs, an optimum computer-aided diagnosis algorithm suitable for the skin map of the electrode placement, and an integrated visual and acoustic alarm system,
- means to analyze, and display data, i.e. a microprocessor based system with a display,
- means for guiding electrode placement and the number of electrodes (11) to be used in related to the severity of the internal organ bleeding, wherein the means for guiding is preferably a display unit (10).

The computer-aided diagnosis system further comprises:
- stimulation means, which comprises an integrated specific electrostimulator and electrodes (11), and
- a connection network to provide electrical power connections and signal connections to all units.

The electrostimulator along with the electrodes (11), display unit (10) and blood pressure measurement and pulse detector unit (13) can also be used solely.

The system can further comprise second input means second input means. Second input means is preferably embedded in a means for guiding and means for guiding is preferably a display unit (10). Said display unit can be a keyboard or a touch screen display, enabling the user, who can be either a person providing a first aid or even the wounded person himself, to enter the wounded or bleeding area information into the system. This information is the location of the wounded or bleeding area.

In case of a touch screen display is used as a means for guiding, second input means can be embedded in the means for guiding, so that the display unit accepts input as well as guides the user where to put the electrodes (11). In this case the user can also pinpoint the location of the wounded or bleeding area on a body drawing or picture appearing on the display.

The skin map for electrode placement contains the location information, where to put the electrodes of the integrated specific electrostimulator, according to the location of the wounded or bleeding area and according to the attributed data.

### Underlying Mechanism of the Proposed invention of decreasing the blood flow and an organ specific skin map:

Segmental receptive field of a multireceptive neurone in the spinal cord is located on the skin and is made up of an excitatory and an inhibitory field. The receptive field very often includes a visceral component and a peculiar convergence of information onto a single neuron occurs [D. Le Bars/Brain Research Reviews 40(2002)29-44]. Sympathetic nerve fibers of the peripheric nerve system are the main target of the internal organ's blood flow decreasing effects that is achieved by high frequency electrostimulation. The stimulation frequency can be between 60 and 90 Hz, preferably at 80 Hz. The pulse duration can be between 0.1-300 us (microseconds).

Percutaneous nerve stimulation or electroacupuncture (EA) shows a very selective action in increasing or decreasing blood flow of a target organ, when appropriate nerve and stimulation frequency combinations are used (Cakmak YO, Akpinar IN, Ekinci G, Bekiroglu N. Point- and frequency-specific response of the testicular artery to abdominal electroacupuncture in humans. Fertil Steril 2008;90:1732-8., Ho M, Huang LC, Chang YY, Chen HY, Chang WC, Yang TC, etal. Electroacupuncture reduces uterine artery blood flow impedance in infertile women. Taiwan J Obstet Gynecol 2009;48:148-51. Stener-Victorin E, Kobayashi R, Watanabe O, Lundeberg T, Kurosawa M. Effect of electroacupuncture stimulation of different frequencies andintensities on ovarian blood flow in anaesthetized rats with steroid-induced polycystic ovaries. Reprod Biol Endocrinol 2004;26:2-16.). We have previously demonstrated (Cakmak YO, Akpinar IN, Ekinci G, Bekiroglu N. Point- and frequency-specific response of the testicular artery to abdominal electroacupuncture in humans. Fertil Steril 2008;90:1732-8.) that blood flow can be increased in the testicular artery of humans with EA by using a specific combination of stimulation frequency and a dermatome, using the effective frequencies that have been proven to increase blood flow in rats by revealing the arterial diameter and area changes in addition to blood flow for the first time in the literature. In the same research, we also demonstrated that closely neighboring dermatomes (T10-T12) have not got the same ability to affect the same organ in means of blood flow.

Ovarian Blood flow-decreasing parameters of electrostimulation had been described in rats (Stener-Victorin E, Kobayashi R, Watanabe O, Lundeberg T, Kurosawa M. Effect of electroacupuncture stimulation of different frequencies and intensities on ovarian blood flow in anaesthetized rats with steroid-induced polycystic ovaries. Reprod Biol Endocrinol 2004;26:2-16.). For the first time, in humans, we demonstrated the optimal blood flow decreasing parameters and dermatome combinations of electrostimulation for human internal organ arteries as the uterine artery in which we decreased the extreme bleeding with a fibroma origin. It has been demonstrated that only high-frequency EA can decrease ovarian blood flow in rats; on the other hand, the neuroanatomical pathways that aid such a function consider central effects in addition to segmental innervation because the ovarian blood flow responses to high frequency EA stimulation were also investigated after severance of the ovarian sympathetic nerves in rats (Stener-Victorin E, Kobayashi R, Watanabe O, Lundeberg T, Kurosawa M. Effect of electroacupuncture stimulation of different frequencies and intensities on ovarian blood flow in anaesthetized rats with steroid-induced polycystic ovaries. Reprod Biol Endocrinol 2004;26:2-16.).

Innervation territories of the single sympathetic C fibers in human skin has different accumulations in different skin zones (J Neurophysiol.1998 Apr; 79(4):1653 60. Innervation territories of single sympathetic C fibers in human skin. Schmelz M, Schmidt R, Bickel A, Torebjörk HE, Handwerker HO, Neurophysiol Clin. 2003 Dec; 33(6):315-24.Trigeminal responses to laser stimuli. Romaniello A, lannetti GD, Truini A, Cruccu G).

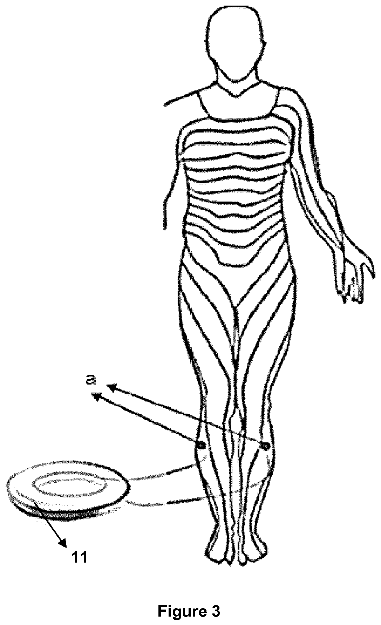

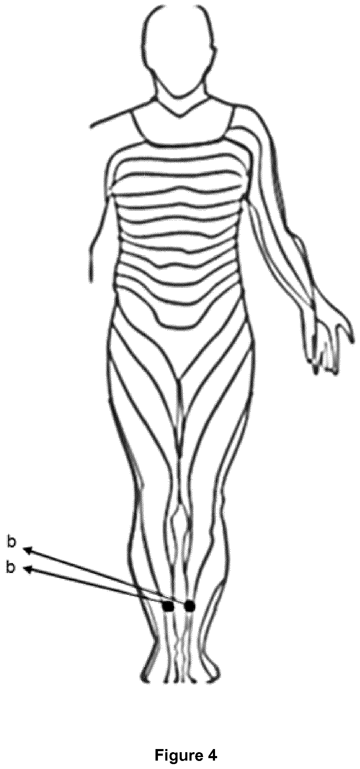

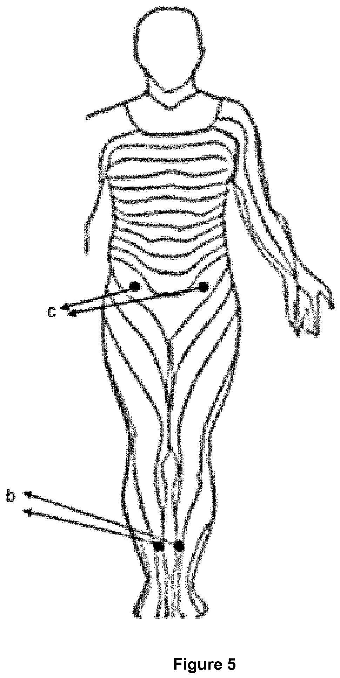

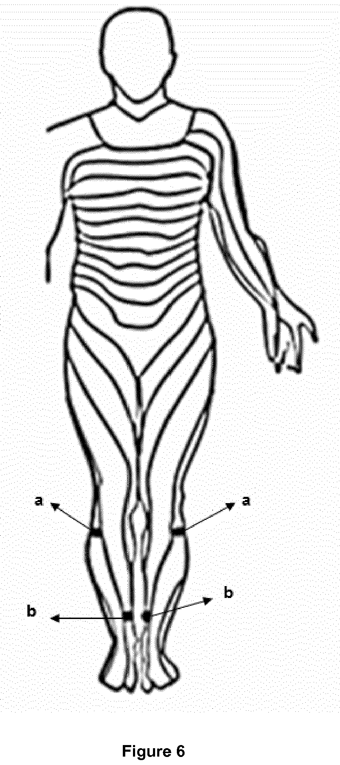

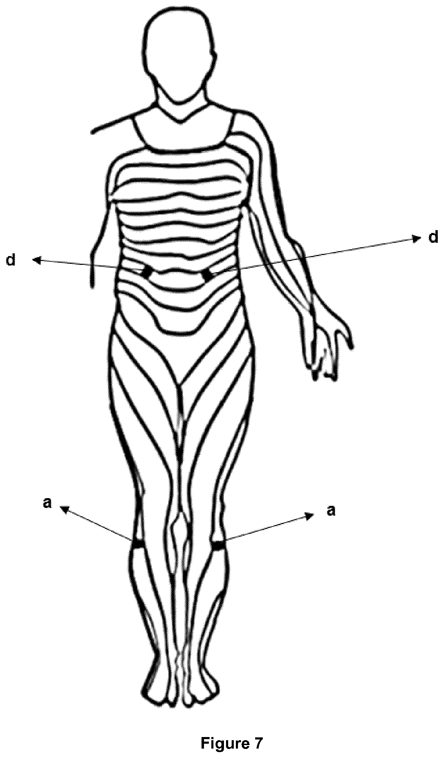

In conclusion, to decrease the blood flow in a specific internal organ needs an optimal combination of stimulation zone of a dermatome and frequency for that specific organ (or organs supplied by the same arterial origin). Bilateral Placement of the 2 electrodes (11) over the L5 dermatome (a) common peroneal nerve territory, anterior to the head of fibular bone impression results with a 20%.decrease of the blood flow to Liver, Stomach, Kidney, Spleen and Proximal Large intestines within 5 minutes (Figure 3). Bilateral placement of the 2 electrodes (11) over the L4-S2 dermatome (b)-saphenous nerve territory, 5-6 cm above the medial malleolus results with a 20% decrease of the blood flow to Distal large intestine, testicles (gonads), Prostate gland and Bladder within 5 min (Figure 4). To achieve 40% blood flow decrease for a specific organ, 4 electrode (11) placements are needed. Bilateral Placement of the 4 electrodes (11) over the L4-S2 dermatomes (b)-saphenous nerve territory, 5-6 cm above the medial malleolus and the T12 dermatome (c) results with a 40% decrease of the blood flow to Distal large intestine, testicles (gonads), Prostate gland and Bladder within 5 min (Figure 5). Bilateral Placement of the 4 electrodes (11) over the L4-S2 dermatomes (b)-saphenous nerve territory, 5-6 cm above the medial malleolus and the L5 dermatome (a)-common peroneal nerve territory-anterior to the head of fibular bone impression or (over the T10 and T12) results with a 40% decrease of the blood flow to all abdominal organs (Figure 6). Bilateral Placement of the 4 electrodes (11) over the L5 dermatome (a) -common peroneal nerve territory-anterior to the head of fibular bone impression and the T10 dermatome (d) decrease blood flow to the Liver, Stomach, Spleen and Proximal Large intestines (Figure 7).

This invention can be more fully understood from the following detailed description when taken in conjunction with the accompanying figures 3 to 7.

## Claims

1. A system to decrease the blood flow of a targeted organ in case of bleeding or a medical requirement, **characterized in that** the system comprises an integrated electrostimulator, which comprises electrodes (11) to be placed to the skin of a patient, and a subsystem which comprises at least one first and at least one second input means, wherein the first input means comprises blood pressure measurement and pulse detector unit and the second input means is a device enabling the user to enter the wounded or bleeding area information into the system, wherein subsystem determines where the said electrodes (11) should be placed according to analyzed blood pressure, pulse and inputted wounded or bleeding area information according to an algorithm embedded in the subsystem, and the subsystem further display where the said electrodes (11) should be placed through at least a skin map.

2. The system according to claim 1 **characterized by** comprising a means for selecting, based on the blood pressure and pulse data, the power of blood decreasing level in the targeted organ/organs.

3. The system according to claim 2 **characterized by** comprising an integrated visual and acoustic alarm system.

4. The system according to claim 1, **characterized by** further comprising a means for high current blocker for currents exceeding 15mA to overcome skin burning.

5. The system according to claim 4, **characterized by** further comprising thermoelectric coolers to cool the skin around the electrodes.

6. The system according claim 1, **characterized in that** said second input means is embedded in a means for guiding.

7. The system according to claim 6 **characterized in that** the means for guiding is preferably a display unit (10).

8. The system according claim 7, **characterized in that** said display unit is a touch screen display.

9. The system according to claim 1, **characterized in that** said electrostimulator, said display unit (10) and said blood pressure measurement and pulse detector unit is used individually.

10. The system according to claim 1, wherein said electrostimulator applies a bipolar electrical signal with stimulation frequency between 60 And 90 Hz and pulse duration between 0.1-300 us.

11. The system according to claim 10, wherein said electrostimulator applies a bipolar electrical signal with stimulation frequency at 80 Hz and pulse duration between 0.1-300 us.

## Patentansprüche

1. System zur Verringerung des Blutflusses eines Zielorgans im Falle einer Blutung oder einer medizinischen Notwendigkeit, **dadurch gekennzeichnet, dass** das System einen integrierten Elektrostimulator, der auf der Haut eines Patienten zu platzierende Elektroden (11) umfasst, und ein Subsystem aufweist, das wenigstens eine erste und wenigstens eine zweite Eingabeeinrichtung umfasst, wobei die erste Eingabeeinrichtung eine Blutdruckmess- und eine Pulsdetektoreinheit beinhaltet und die zweite Eingabeeinrichtung ein Gerät ist, das es dem Bediener ermöglicht, Gebietsinformation der Wunde oder der Blutung in das System einzugeben, wobei das Subsystem bestimmt, wo die Elektroden (11) gemäß dem analysierten Blutdruck, Puls und der eingegebenen Gebietsinformation der Wunde oder der Blutung auf Grundlage eines in dem Subsystem eingebetteten Algorithmus platziert sollten, und wobei das Subsystem weiter über wenigstens eine Hautkarte anzeigt, wo die Elektroden (11) platziert werden sollten.

2. System nach Anspruch 1, **dadurch gekennzeichnet, dass** es eine Einrichtung zum Auswählen, auf Grundlage der Blutdruck- und Pulsdaten, des Umfangs der Blutflussverringerung in dem/den Zielorgan/Zielorganen aufweist.

3. System nach Anspruch 2, **dadurch gekennzeichnet, dass** es ein integriertes visuelles und akustisches Alarmsystem aufweist.

4. System nach Anspruch 1, **dadurch gekennzeichnet, dass** es weiter einen Hochstromblocker für Ströme, die 15 mA übersteigen, aufweist, um Hautverbrennungen zu vermeiden.

5. System nach Anspruch 4, **dadurch gekennzeichnet, dass** es weiter thermoelektrische Kühler aufweist, um die Haut um die Elektroden herum zu kühlen.

6. System nach Anspruch 1, **dadurch gekennzeichnet, dass** die zweite Eingabeeinrichtung in eine Führungseinrichtung eingebettet ist.

7. System nach Anspruch 6, **dadurch gekennzeichnet, dass** die Führungseinrichtung vorzugsweise eine Anzeigeeinheit (10) ist.

8. System nach Anspruch 7, **dadurch gekennzeichnet, dass** die Anzeigeeinheit eine Touchscreen-Anzeige ist.

9. System nach Anspruch 1, **dadurch gekennzeichnet, dass** der Elektrostimulator, die Anzeigeeinheit (10) und die Blutdruckmess- und Pulsdetektoreinheit individuell verwendet werden.

10. System nach Anspruch 1, wobei der Elektrostimulator ein bipolares elektrisches Signal mit einer Stimulationsfrequenz zwischen 60 und 90 Hz und einer Pulsdauer zwischen 0,1 - 300 µs abgibt.

11. System nach Anspruch 10, wobei der Elektrostimulator ein bipolares elektrisches Signal mit einer Stimulationsfrequenz bei 80 Hz und einer Pulsdauer zwischen 0,1 - 300 µs abgibt.

## Revendications

1. Système pour diminuer le flux sanguin d'un organe ciblé en cas de saignement ou d'une exigence médicale, **caractérisé en ce que** le système comprend un électrostimulateur intégré, qui comprend des électrodes (11) à placer sur la peau d'un patient, et un sous-système qui comprend au moins un premier et au moins un second moyen d'entrée, dans lequel le premier moyen d'entrée comprend une unité de mesure de pression sanguine et de détection de pulsations et le second moyen d'entrée est un dispositif permettant à l'utilisateur de saisir les informations de la zone de lésion ou de saignement dans le système, dans lequel un sous-système détermine où lesdites électrodes (11) doivent être placées selon une pression sanguine, une pulsation et des informations de zone de lésion ou de saignement entrées analysées selon un algorithme incorporé dans le sous-système, et le sous-système affiche en outre où lesdites électrodes (11) doivent être placées via au moins une carte de peau.

2. Système selon la revendication 1 **caractérisé en ce qu'**il comprend un moyen pour sélectionner, sur la base de données de pression sanguine et de pulsations, la puissance de niveau de diminution de sang dans l'organe/les organes ciblé(s).

3. Système selon la revendication 2 **caractérisé en ce qu'**il comprend un système d'alarme visuelle et acoustique intégré.

4. Système selon la revendication 1, **caractérisé en ce qu'**il comprend en outre un moyen pour un bloqueur de courant élevé pour des courants dépassant 15 mA pour surmonter des brûlures cutanées.

5. Système selon la revendication 4, **caractérisé en ce qu'**il comprend en outre des refroidisseurs thermoélectriques pour refroidir la peau autour des électrodes.

6. Système selon la revendication 1, **caractérisé en ce que** ledit second moyen d'entrée est incorporé dans un moyen de guidage.

7. Système selon la revendication 6, **caractérisé en ce que** le moyen de guidage est de préférence une unité d'affichage (10).

8. Système selon la revendication 7, **caractérisé en ce que** ladite unité d'affichage est un dispositif d'affichage à écran tactile.

9. Système selon la revendication 1, **caractérisé en ce que** ledit électrostimulateur, ladite unité d'affichage (10) et ladite unité de mesure de pression sanguine et de détection de pulsations sont utilisés individuellement.

10. Système selon la revendication 1, dans lequel ledit électrostimulateur applique un signal électrique bipolaire avec une fréquence de stimulation entre 60 et 90 Hz et une durée de pulsation entre 0,1 et 300 µs.

11. Système selon la revendication 10, dans lequel ledit électrostimulateur applique un signal électrique bipolaire avec une fréquence de stimulation à 80 Hz et une durée de pulsation entre 0,1 et 300 µs.
